# EUROPEAN PATENT APPLICATION

(11) **EP 3 560 549 A1**
(43) Date of publication of application: **30.10.2019**
(21) Application number: 16924896.0
(22) Date of filing: 20.12.2016
(51) Int. Cl.: A61N 1/05, H01B 1/02

(54) **CARDIAC PACING CURRENT-PROVIDING WIRE, UNIT AND DEVICE**

(71) Applicant: Nanjing Xin Pai Medical Technology Co., Ltd, Nanjing, Jiangsu 210000 (CN)
(72) Inventor: YU, Shuping, Nanjing Jiangsu 210000 (CN); ZHANG, Hao, Shanghai 200433 (CN)
(74) Representative: Taor, Simon Edward William
(86) International application number: PCT/CN2016/111169
(87) International publication number: WO 2018/112767

(57) **Abstract**

A degradable cardiac pacing current-providing wire, comprising: conductive core; insulating film ,covering said conductive core, wherein said conductive core is made of magnesium alloy, the weight percentage of said magnesium alloy is: zinc 4.5-5.5%, manganese 0.8-1.2%, calcium 0.8-1.2%, and the remainder is magnesium, said insulating film is made of either or at least two copolymers of Polylactic acid (PLA), Polyglycolic acid (PGA), Polycaprolactone (PCL), Polydioxanone (PDS) and Polyhydroxyalkanoate (PHA). A cardiac pacing current-providing unit, for use in providing pacing current to the heart of the patient, comprising: wire; suture needle and connecting electrode which is connected to the cardiac pacemaker, there is no wire residue in the body after degradation, and no metal artifacts will occur, which makes the patients free from the need for re-operation, and reduces the mental, physical and economic burden caused by the operation, has a strong clinical application value.

## Description

### FIELD OF THE INVENTION

The present invention relates to the field of cardiac pacing and belongs to the field of medical devices.

### BACKGROUND OF THE INVENTION

For patients with transient bradycardia (such as bradycardia after open heart surgery), temporary pacemaker leads are often implanted surgically to improve heart rate and cardiac output early after surgery and accelerate the recovery process.

However, the existing temporary pacemaker leads are made of non-degradable medical polymer materials and stainless steel wire, patients do not need to continue pacing treatment after their heart rhythm returns to normal, temporary pacemaker leads are permanently stored in patients. They can be clearly seen during routine chest X-ray examinations such as work physical examination and pre-marital physical examination, which has a serious impact on patients' lives.

In addition, the existing temporary pacemaker leads need to be fixed in the myocardium by manual knotting, which not only makes the operation cumbersome, but also causes different degrees of myocardial damage.

### SUMMARY OF THE INVENTION

The present invention is made for solving the above problems and aims to provide a degradable cardiac pacing current-providing wire. After implantation, the cardiac pacing current-providing wire of the present invention degrades sequentially from outside to inside through chemical and electrochemical reactions with tissue fluid. The degrading product can be absorbed and utilized by human body or discharged with urine in the form of metabolites without surgery, thus effectively solving the above problems.

In addition, The cardiac pacing current-providing wire of the present invention can be directly anchored on the epicardium without manual knotting and fixation, which not only shortens the operation time, but also reduces the damage to the myocardial tissue.

The cardiac pacing current-providing wire of the present invention, one end is fixed to the epicardium and myocardial tissue, and the other end is connected to a cardiac pacemaker placed outside the heart for use in providing pacing current to the heart of the patient, can have the following features: conductive core; insulating film ,covering the conductive core, wherein the conductive core is made of magnesium alloy, the weight percentage of the magnesium alloy is: zinc 4.5-5.5%, manganese 0.8-1.2%, calcium 0.8-1.2%, and the remainder is magnesium, the insulating film is made of either or at least two copolymers of Polylactic acid (PLA), Polyglycolic acid (PGA), Polycaprolactone (PCL), Polydioxanone (PDS) and Polyhydroxyalkanoate (PHA).

Further, the cardiac pacing current-providing wire of the present invention can also have the following features: wherein the conductive core is composed of a single magnesium alloy wire or a plurality of magnesium alloy wires.

The cardiac pacing current-providing unit of the present invention, for use in providing pacing current to the heart of the patient, can have the following features: wire; suture needle, arranged at one end of the wire for penetrating the heart muscle and fixing the wire on the epicardium and myocardial tissue; connecting electrode, arranged at the other end of the wire, is connected to the cardiac pacemaker, wherein the wire is any kind of the above cardiac pacing current-providing wire.

Further, the cardiac pacing current-providing unit of the present invention can also have the following features: anti-separation member, arranged at the end portion of one end connected with the suture needle on the wire, and the material of the anti-separation member is the same as that of the insulating film or the conductive core, after the anti-separation member guides the conductive core through the epicardium and myocardial tissue, pulling back the wire can anchor the anti-separation member on the epicardium to avoid slipping.

Further, the cardiac pacing current-providing unit of the present invention can also have the following features: wherein the anti-separation member is a barb or a triangular block gradually reduced in the direction of the suture needle.

Further, the cardiac pacing current-providing unit of the present invention can also have the following features: wherein the barb is a flexible convex strip ,and the convex strip is formed by cutting the linear segment portion of the conductive core, or by welding or bonding on the linear segment portion, with a length of 2-10 mm and a thickness of 0.1-1 mm.

Further, the cardiac pacing current-providing unit of the present invention can also have the following features: wherein the barb is formed by cutting the conductive core, or is processed with a magnesium alloy material identical to the conductive core, and then welded or bonded to the conductive core.

Further, the cardiac pacing current-providing unit of the present invention can also have the following features: wherein the suture needle is a semicircular suture needle, and the tail end of the suture needle is provided with a fixed slot for occluding and fixing the conductive core, the barb is an arc barb bending towards the suture needle.

Further, the cardiac pacing current-providing unit of the present invention can also have the following features: wherein the connecting electrode is a straight needle with a depression, the straight needle is used to directly pierce the chest wall and expose the body, the depression is convenient for cutting or breaking the straight needle.

The cardiac pacing current-providing device of the present invention can have the following features: at least one cardiac pacing current-providing unit and a cardiac pacemaker connected with the cardiac pacing current-providing unit, wherein the cardiac pacing current-providing unit is any kind of the above cardiac pacing current-providing unit

### THE EFFECT OF THE PRESENT INVENTION

According to cardiac pacing current-providing wire of the present invention, the conductive core of the cardiac pacing current-providing wire is a degradable magnesium alloy material and the insulating film is a degradable organic macromolecule material, so the cardiac pacing current-providing wire can be degraded sequentially from outside to inside within a certain time after implantation: the outer insulating film first degrades into a non-toxic normal metabolite of the human body, and then the magnesium alloy of the conductive core contacted with the tissue solution and degraded into soluble electrolyte by hydrolysis reaction. Some of the degradation products are absorbed and utilized by human body, while others are excreted with urine through the urinary system. Therefore, there is no wire residue in the body after degradation, and no metal artifacts will occur, which makes the patients free from the need for re-operation, and reduces the mental, physical and economic burden caused by the operation, has a strong clinical application value.

### BRIEF DESCRIPTION OF THE DRAWINGS

FIG1 is the structure schematic diagram of the cardiac pacing current-providing unit in embodiment 1 of the present invention;
FIG2 is the structure schematic diagram of the suture needle in embodiment 1 of the present invention, FIG2a is the structure schematic diagram of the barb, FIG2b is the enlarged schematic diagram of the connection between the suture needle and the exposed wire through the fixed slot, FIG2c is the structure schematic diagram of the barb which is formed by cutting the conductive core, FIG2d is the structure schematic diagram of the barb which is welding or bonding on the conductive core;
FIG3 is the structural profile of the the cardiac pacing current-providing wire which is consisting of a single magnesium alloy conductor and insulating film in embodiment 1 of the present invention;
FIG4 is the schematic diagram of the anchor method in embodiment 1 of the present invention, FIG4a is the schematic diagram of the suture needle penetrating epicardium and myocardial tissue, FIG4b is the schematic diagram of the back stab anchored to the epicardium after pulling back the wire;
FIG5 is the structure schematic diagram of the triangular block in embodiment 2 of the present invention, FIG5a is the structure schematic diagram of the wedge block of triangular, FIG5b is the structure schematic diagram of the wedge block with back depression;
FIG6 is the structural profile of the the cardiac pacing current-providing wire which is consisting of multiple magnesium alloy conductor and insulating film in embodiment 2 of the present invention;
FIG7 is the schematic diagram of the anchor method in embodiment 2 of the present invention, FIG4a is the schematic diagram of the suture needle penetrating epicardium and myocardial tissue, FIG4b is the schematic diagram of the back stab anchored to the epicardium after pulling back the wire.

### DETAILED DESCRIPTION OF THE PREFERRED EMBODIMENTS

### <Embodiment 1>

The cardiac pacing current-providing device in embodiment 1, comprising two cardiac pacing current-providing units 100 and a cardiac pacemaker connected with said cardiac pacing current-providing unit 100. The cardiac pacemaker is placed outside the the body of patients with heart disease for use in providing pacing current to the heart of the patient

The degradable cardiac pacing current-providing unit 100 in embodiment 1, one end is fixed to the epicardium, and the other end is connected to a cardiac pacemaker placed outside the heart.

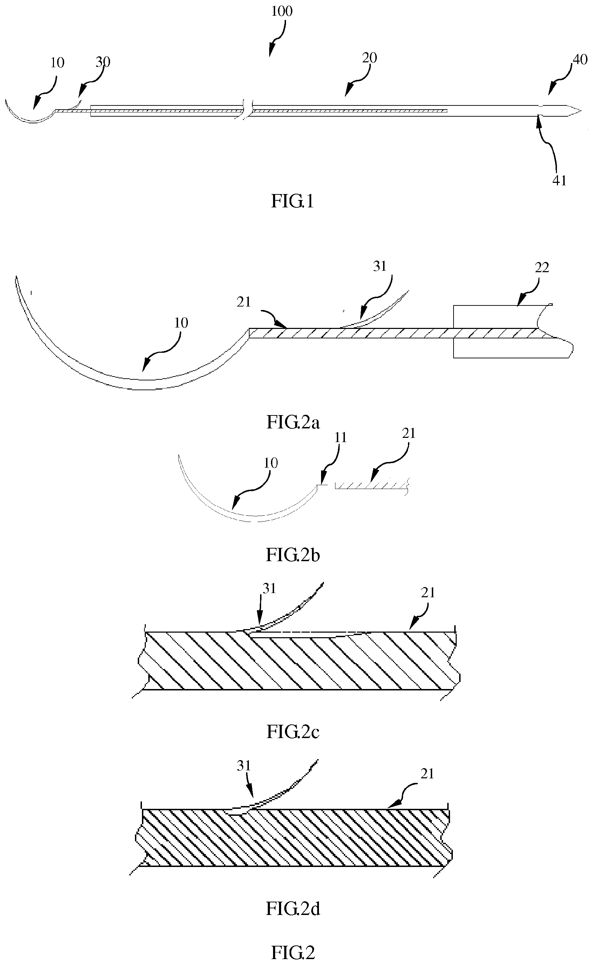

FIG1 is the structure schematic diagram of the cardiac pacing current-providing unit in embodiment 1 of the present invention.

As shown in FIG1, the degradable cardiac pacing current-providing unit 100 comprising sequentially connected suture needle 10, cardiac pacing current-providing wire 20, anti-separation member 30 and connecting electrode 40.

FIG2 is the structure schematic diagram of the suture needle in embodiment 1 of the present invention, FIG2a is the structure schematic diagram of the barb, FIG2b is the enlarged schematic diagram of the connection between the suture needle and the exposed wire through the fixed slot, FIG2c is the structure schematic diagram of the barb which is formed by cutting the conductive core, FIG2d is the structure schematic diagram of the barb which is welding or bonding on the conductive core

As shown in FIG2a and FIG2b, suture needle 10 is for using in penetrating the epicardium and myocardial tissue, located at the front end, is a semicircular stainless steel medical suture needle, and the tail end of the suture needle is a fixed slot 11 for using in occluding and fixing the conductive core, as shown in FIG2b.

The cardiac pacing current-providing wire 20 comprising conductive core 21 and insulating film 22 which is for using in covering the conductive core 21.

The anti-separation member 30, is a barb 31 in embodiment 1, as shown in FIG2a, the barb 31 an arc barb bending towards the suture needle 10.

The exposed conductive core 21 and the barb 31 are made of degradable magnesium alloy in human body.

As shown in FIG2c, the barb 31 is formed by cutting the conductive core 21, this kind of processing method is relatively fast and suitable for rapid mass production. In order to ensure the strength of the barbs, the bars processed are 2-10 mm in length and 0.1-1.0 mm in thickness.

As shown in FIG2d, the barb 31 is also can be the degradable magnesium alloy wire which is welded or bonded on the conductive core 21.

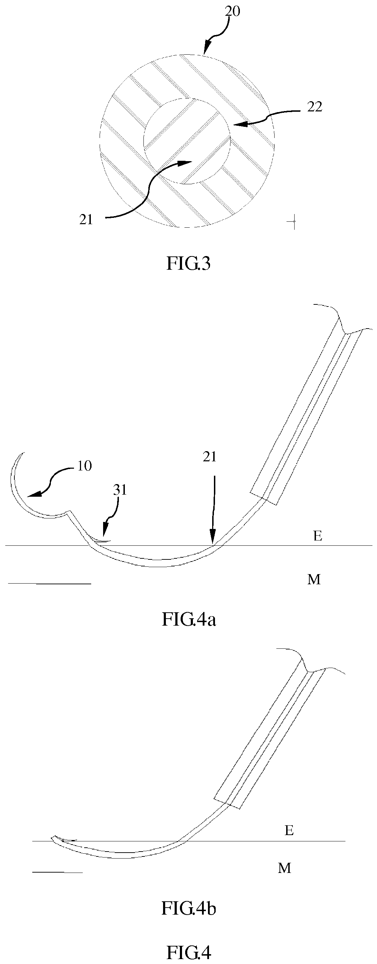

The detailed structure of the cardiac pacing current-providing wire 20 is illustrated below in conjunction with Fig. 3.

FIG3 is the structural profile of the the cardiac pacing current-providing wire which is consisting of a single magnesium alloy conductor and insulating film in embodiment 1 of the present invention.

The cardiac pacing current-providing wire 20, as shown in FIG1 and FIG3, comprising the degradable conductive core 21 in the human body and the degradable insulating film 22 which is for using in covering the conductive core 21.

The material of the conductive core 21 is magnesium alloy, the weight percentage of the magnesium alloy is: zinc 4.5%, manganese 0.8%, calcium 0.8%, and the remainder 96.4% is magnesium. In embodiment 1, the exposed conductive core 21 and the barb 31 have the same material, and they are the same magnesium alloy wire.

The insulating film 22 is medical grade copolymer of Polylactic acid (PLA) and Polydioxanone (PDS),which is covering the whole conductive core 21.

As shown in FIG1, the connecting electrode 40 is a straight needle with a depression 41, said straight needle is used to directly pierce the chest wall and expose the body, said depression 41 is convenient for cutting or breaking said straight needle.

The connecting electrode 40 is made of stainless steel. The left end of the connecting electrode 40 shown as the FIG is connected with the conductive core 21 which is exposed insulating film 22, and the right end is pointed free tip.

FIG4 is the schematic diagram of the anchor method in embodiment 1 of the present invention, FIG4a is the schematic diagram of the suture needle penetrating epicardium and myocardial tissue, FIG4b is the schematic diagram of the back stab anchored to the epicardium after pulling back the wire.

The following is a detailed description of the method of using the cardiac pacing current-providing wire in embodiment 1 in conjunction with FIG 4:
During the operation, using the suture needle 11 to guide the barb of the cardiac pacing current-providing wire through the epicardium and myocardial tissue of the atrium or ventricle, as shown in Fig. 4a. Then, the semicircular stainless steel medical suture needle was cut off and the cardiac pacing current-providing wire was pulled back so that the barb 31 was tightly fixed on the epicardium to avoid slipping, instead of knotting and fixing in routine surgery, which shortened the operation time and reduced the myocardial damage.

Then, using the free tip of the connecting electrode 40 to guide the tail end of the cardiac pacing current-providing wire to pierce through the chest wall to expose the body, and a part of the connecting electrode 40 is removed from the depression 41, and the remaining part is used as a metal electrode to connect with the cardiac pacemaker outside the patient's body.

When in use, the cardiac pacemaker sends a pacemaker pulse current into the atrium or ventricular muscle for pacing by connecting connecting electrode 40, cardiac pacing current-providing wire 20 and barb 31.

The degradation process of the cardiac pacing current-providing wire in embodiment 1 is as follows:
Firstly, the insulating film on the outer layer of conductive core 21 has good biocompatibility and initial mechanical properties, under the action of the tissue solution in the human body, it will hydrolyze or enzymatic hydrolysis within a set time to become non-toxic normal metabolites of human body, such as carbon dioxide and water, and will be discharged with urine. When the insulating film on the outer layer of conductive core 21 is degraded, the conductive core of the metal is exposed to the weak acidic tissue solution (pH 6.7). On the one hand, the potential of each metal in the magnesium alloy is different, and electrochemical degradation occurs, which makes the metal degrade into metal ions and dissolve in the tissue solution, on the other hand, formed soluble electrolytes by hydrolysis reaction with water in the tissue solution.

### THE EFFECT OF EMBODIMENT 1

According to the cardiac pacing current-providing unit in embodiment 1, the conductive core of the cardiac pacing current-providing wire is a degradable magnesium alloy material and the insulating film is a degradable organic macromolecule material, so the cardiac pacing current-providing wire can be degraded sequentially from outside to inside within a certain time after implantation: the outer insulating film first degrades into a non-toxic normal metabolite of the human body, and then the magnesium alloy of the conductive core contacted with the tissue solution and degraded into soluble electrolyte by hydrolysis reaction. Some of the degradation products are absorbed and utilized by human body, while others are excreted with urine through the urinary system. Therefore, there is no wire residue in the body after degradation, and no metal artifacts will occur, which makes the patients free from the need for re-operation, and reduces the mental, physical and economic burden caused by the operation, has a strong clinical application value.

Further, since the barb in embodiment 1 has the function of preventing slippage, when used, the barb passes through the epicardium and myocardial tissue under the guidance of the suture needle, and barb pulling the cardiac pacing current-providing wire can fix the barb on the epicardium, which not only effectively prevents the cardiac pacing current-providing wire from prolapsing in the myocardial tissue, but also does not need manual knotting and fixing in the operation process, thus shortening the operation time and reducing the myocardial damage.

Furthermore, since the connecting electrode is a straight needle with annular depression, it is easy to remove the straight needle from the depression after piercing the chest wall and exposing the body, the tail end of the straight needle can be directly connected with the positive or negative pole of the cardiac pacemaker, which further facilitates the operation of doctors and shortens the operation time.

### <Embodiment 2>

In embodiment 2, the same structure is given the same number as in embodiment 1.

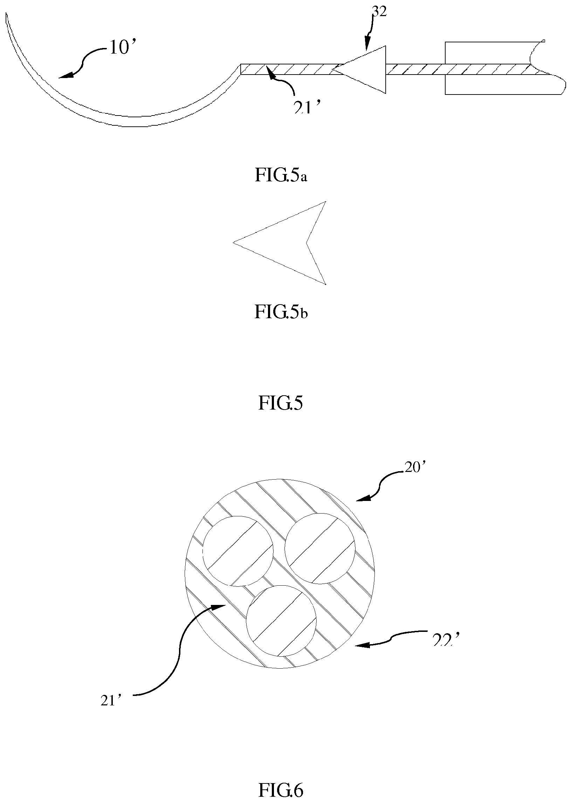

FIG5 is the structure schematic diagram of the triangular block in embodiment 2 of the present invention, FIG5a is the structure schematic diagram of the wedge block of triangular, FIG5b is the structure schematic diagram of the wedge block with back depression.

FIG6 is the structural profile of the the cardiac pacing current-providing wire which is consisting of multiple magnesium alloy conductor and insulating film in embodiment 2 of the present invention.

In embodiment 2, it provides an alternative to the barb 31 in embodiment 1 by using a triangular block 32, and provides an alternative to the conductive core 21 woven by single magnesium alloy wire by using a conductive core 21' woven by three magnesium alloy wires.

As shown in Fig.5, the suture needle 10', the exposed conductive core 21 and the triangular block 32 which is arranged on the conductive core 21 and is gradually reduced towards the suture needle 10'. The exposed conductive core 21' is made of degradable magnesium alloy material in human body. The weight percentage of the magnesium alloy is: zinc 5.5%, manganese 1.2%, calcium 1.2%, strontium 2% and the remainder 90.1% is magnesium. The material of the triangular block 32 is also degradable material, which is the same as that of insulating film.

The triangular block 32 is arranged around the exposed conductive core 21, with the free tip facing the suture needle 10'. As shown in Fig.5a and Fig.5b, the triangular block 32 may be a triangular block shown in Fig.5a or a triangular block with a depression at the back shown in Fig.5b.

As shown in Fig.6, the conductive core 21' is woven or twisted by three magnesium alloy wires with the same composition as the exposed conductive core 21.

Insulating film 22' is medical grade copolymer of Polylactic acid (PLA) and Polyglycolic acid (PGA),which is covering the whole conductive core 21'.

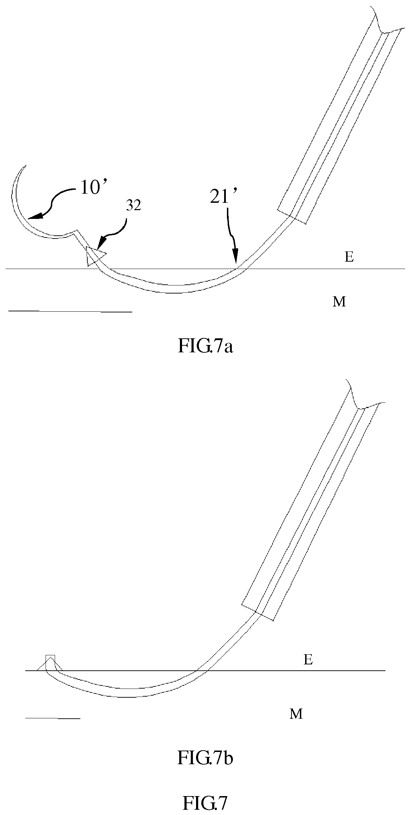

FIG7 is the schematic diagram of the anchor method in embodiment 2 of the present invention, FIG4a is the schematic diagram of the suture needle penetrating epicardium and myocardial tissue, FIG4b is the schematic diagram of the back stab anchored to the epicardium after pulling back the wire.

As shown in Fig.7a and Fig.7b, after the exposed conductive core 21' is guided by the suture needle 10' through the myocardial tissue M and epicardium E, the triangular block 32 is fixed on the epicardium, thus avoiding the knotting and fixing in routine surgery.

### THE EFFECT OF EMBODIMENT 2

According to the cardiac pacing current-providing unit in embodiment 2, since the triangular block on the suture needle in embodiment 2 has the function of preventing detachable, there is no need to knot and fix during the operation, which shortens the operation time and reduces the myocardial damage.

Further, since the conductive core is a braided structure of three alloy wires, the strength is higher.

## Claims

1. A cardiac pacing current-providing wire, one end is fixed to the epicardium and myocardial tissue, and the other end is connected to a cardiac pacemaker placed outside the heart for use in providing pacing current to the heart of the patient, comprising:
conductive core;
insulating film ,covering said conductive core,
wherein said conductive core is made of magnesium alloy, the weight percentage of said magnesium alloy is: zinc 4.5-5.5%, manganese 0.8-1.2%, calcium 0.8-1.2%, and the remainder is magnesium,
said insulating film is made of either or at least two copolymers of polylactic acid (PLA), polyglycolic acid (PGA), polycaprolactone (PCL), polydioxanone (PDS) and polyhydroxyalkanoate (PHA).

2. The cardiac pacing current-providing wire according to claim 1,
wherein said conductive core is composed of a single magnesium alloy wire or a plurality of magnesium alloy wires.

3. A cardiac pacing current-providing unit, for use in providing pacing current to the heart of the patient, comprising:
wire;
suture needle, arranged at one end of said wire for penetrating the heart muscle and fixing said wire on the epicardium and myocardial tissue;
connecting electrode, arranged at the other end of said wire, connected to the cardiac pacemaker,
wherein said wire is any kind of the cardiac pacing current-providing wire in claims 1∼2.

4. The cardiac pacing current-providing unit according to claim 3, comprising:
anti-separation member, arranged at the end portion of one end connected with said suture needle on said wire, and the material of said anti-separation member is the same as that of said insulating film or said conductive core,
after said anti-separation member guides the conductive core through the epicardium and myocardial tissue, pulling back said wire can anchor said anti-separation member on the epicardium to avoid slipping.

5. The cardiac pacing current-providing unit according to claim 3,
wherein said anti-separation member is a barb or a triangular block gradually reduced in the direction of said suture needle.

6. The cardiac pacing current-providing unit according to claim 5,
wherein said barb is a flexible convex strip ,and said convex strip is formed by cutting the linear segment portion of said conductive core, or by welding or bonding on said linear segment portion, with a length of 2-10 mm and a thickness of 0.1-1 mm.

7. The cardiac pacing current-providing unit according to claim 5,
wherein said barb is formed by cutting said conductive core, or is processed with a magnesium alloy material identical to said conductive core, and then welded or bonded to said conductive core.

8. The cardiac pacing current-providing unit according to claim 4,
wherein said suture needle is a semicircular suture needle, and the tail end of said suture needle is provided with a fixed slot for occluding and fixing said conductive core,
said barb is an arc barb bending towards said suture needle.

9. The cardiac pacing current-providing unit according to claim 3,
wherein said connecting electrode is a straight needle with a depression, said straight needle is used to directly pierce the chest wall and expose the body, said depression is convenient for cutting or breaking said straight needle.

10. A cardiac pacing current-providing device, comprising:
at least one cardiac pacing current-providing unit and a cardiac pacemaker connected with said cardiac pacing current-providing unit,
wherein said cardiac pacing current-providing unit is any kind of the cardiac pacing current-providing unit in claims 3∼9.
